# EUROPEAN PATENT APPLICATION

(11) **EP 4 437 971 A1**
(43) Date of publication of application: **02.10.2024**
(21) Application number: 22898629.5
(22) Date of filing: 24.11.2022
(51) Int. Cl.: A61B 10/00, A61B 5/00, A61B 5/02, A61B 5/16, A61B 5/352

(54) **MENTAL DISEASE DETERMINATION METHOD, PROGRAM, MENTAL DISEASE DETERMINATION APPARATUS, AND MENTAL DISEASE DETERMINATION SYSTEM**

(30) Priority: 25.11.2021 JP 2021191250
(71) Applicant: Sumitomo Pharma Co., Ltd., Osaka 541-0045 (JP); Kinki University, Higashiosaka-shi, Osaka 577-8502 (JP)
(72) Inventor: KAWARAI, Hiroko, Osaka-shi, Osaka 554-0022 (JP); IKEDA, Kazuhito, Suita-shi, Osaka 564-0053 (JP); IWAMURA, Yoshihiro, Suita-shi, Osaka 564-0053 (JP); KATSURA, Yasunori, Suita-shi, Osaka 564-0053 (JP); IWASAKI, Tsuyoshi, Osaka-shi, Osaka 554-0022 (JP); ICHIKAWA, Osamu, Osaka-shi, Osaka 554-0022 (JP); YANAGI, Masaya, Osakasayama-shi, Osaka 589-8511 (JP)
(74) Representative: Mewburn Ellis LLP
(86) International application number: PCT/JP2022/043420
(87) International publication number: WO 2023/095847

(57) **Abstract**

A determination method for a psychiatric disorder includes: acquiring a first reference value, a second reference value, a first comparison value, and a second comparison value, the first reference value being a heart rate variability index of a healthy person under a first sound stimulus environment, the second reference value being a heart rate variability index of the healthy person under a second sound stimulus environment different from the first sound stimulus environment, the first comparison value being a heart rate variability index of a user under the first sound stimulus environment, the second comparison value being a heart rate variability index of the user under the second sound stimulus environment; and executing comparison processing among the first reference value, the second reference value, the first comparison value, and the second comparison value to determine a possibility that the user has a psychiatric disorder.

## Description

### TECHNICAL FIELD

The present invention relates to a determination method for a psychiatric disorder, a program, a determination device for a psychiatric disorder, and a determination system for a psychiatric disorder.

### BACKGROUND ART

Patent Document 1 discloses a mental and physical state determination device including a sound output unit for making a subject listen to environmental music and a video output unit for showing an environmental video to the subject.

Patent Document 2 discloses a mental state determination method for determining depression of a user using medical questionnaire data and measurement data obtained by measuring changes in a high-frequency component (HF) and a low-frequency component (LF) of the user to whom mental load is applied.

### PRIOR ART DOCUMENT

### PATENT DOCUMENT

Patent Document 1: JP 6233776 B
Patent Document 2: JP 6635507 B

### SUMMARY OF THE INVENTION

### PROBLEM TO BE SOLVED BY THE INVENTION

In the mental and physical state determination device of Patent Document 1, it is necessary to measure pulse waves before and after the device shows a video to the subject and lets the subject to listen to music. Further, it is necessary to display an instruction on the video output unit and make the subject perform an operation. Therefore, the mental and physical state determination device of Patent Document 1 may not be easily used.

In the depressed state determination method of Patent Document 2, it is necessary to acquire medical questionnaire data, and to measure the changes in a heart rate variability index for before, during, and after application of a mental load to a user. Therefore, the depressed state determination method of Patent Document 2 may not be easily used.

An object of the present disclosure is to provide a determination method for a psychiatric disorder, a program, a psychiatric disorder determination, and a determination system for a psychiatric disorder that can be used more easily.

### SOLUTION TO THE PROBLEM

An aspect of the present disclosure provides a determination method for a psychiatric disorder, including:
acquiring a first reference value, a second reference value, a first comparison value, and a second comparison value, the first reference value being a heart rate variability index of a healthy person under a first sound stimulus environment, the second reference value being a heart rate variability index of the healthy person under a second sound stimulus environment different from the first sound stimulus environment, the first comparison value being a heart rate variability index of a user under the first sound stimulus environment, the second comparison value being a heart rate variability index of the user under the second sound stimulus environment; and
executing comparison processing among the first reference value, the second reference value, the first comparison value, and the second comparison value to determine a possibility that the user has a psychiatric disorder.

An aspect of the present disclosure provides a program for causing a computer to execute the determination method according to the above aspect.

An aspect of the present disclosure provides a determination device for a psychiatric disorder, including:
an acquisition unit configured to acquire a first reference value, a second reference value, a first comparison value, and a second comparison value, the first reference value being a heart rate variability index of a healthy person under a first sound stimulus environment, the second reference value being a heart rate variability index of the healthy person under a second sound stimulus environment, the first comparison value being a heart rate variability index of a user under the first sound stimulus environment, the second comparison value being a heart rate variability index of the user under the second sound stimulus environment; and
a determination unit configured to determine a possibility that the user has a psychiatric disorder based on the first reference value, the second reference value, the first comparison value, and the second comparison value that are acquired.

An aspect of the present disclosure provides a determination system for a psychiatric disorder, including:
the determination device according to the above aspect, and
an analysis device configured to analyze a heart rate variability index of the healthy person or the user; and
a stimulation device configured to give a sound stimulus to the healthy person or the user.

### ADVANTAGEOUS EFFECT OF THE INVENTION

The determination method according to the above aspect can achieve a determination method for a psychiatric disorder that can be used more easily.

The program according to the above aspect can cause the computer to execute the determination method for a psychiatric disorder that can be used more easily.

The determination device according to the above aspect can achieve a determination device for a psychiatric disorder that can be used more easily.

The determination system according to the above aspect can achieve by the determination device a determination system for a psychiatric disorder that can be used more easily.

### BRIEF DESCRIPTION OF THE DRAWINGS

Fig. 1 is a block diagram illustrating a determination system for a psychiatric disorder according to an embodiment of the present disclosure.
Fig. 2 is a graph illustrating an example of low frequency (LF)/high frequency (HF) of healthy persons.
Fig. 3 is a graph illustrating an example of LF/HF of patients with bipolar I disorder.
Fig. 4 is a graph illustrating an example of LF/HF of patients with schizophrenia.
Fig. 5 is a flowchart for explaining an example of a determination method for a psychiatric disorder of the present disclosure.
Fig. 6 is a graph illustrating an example of LF/HF of healthy persons.
Fig. 7 is a graph illustrating an example of LF/HF of depressed patients.
Fig. 8 is a graph illustrating an example of LF/HF of patients with bipolar disorder.
Fig. 9 is a graph illustrating an example of LF/HF of patients with bipolar I disorder.
Fig. 10 is a graph illustrating an example of LF/HF of patients with bipolar II disorder.
Fig. 11 is a graph illustrating an example of LF/HF of patients with schizophrenia.
Fig. 12 is a graph illustrating an example of LF/HF of healthy persons.
Fig. 13 is a graph illustrating an example of LF/HF of depressed patients.
Fig. 14 is a graph illustrating an example of LF/HF of patients with bipolar disorder.
Fig. 15 is a graph illustrating an example of LF/HF of patients with bipolar I disorder.
Fig. 16 is a graph illustrating an example of LF/HF of patients with bipolar II disorder.
Fig. 17 is a graph illustrating an example of LF/HF of patients with schizophrenia.
Fig. 18 is a graph illustrating an example of an analysis result using a combination of LF/HF and HF.
Fig. 19 is a graph illustrating an example of an analysis result using a combination of HF and HR.

### DETAILED DESCRIPTION

Hereinafter, an example of the present disclosure will be described with reference to the accompanying drawings. In the following description, terms (terms including, for example, "upper", "lower", "right", "left", etc.) indicating specific directions or positions are used as necessary, but the use of these terms is to facilitate understanding of the present disclosure with reference to the drawings, and the technical scope of the present disclosure is not limited by the meanings of these terms. Further, the following description is essentially an example, and is not intended to limit the present disclosure, its application, or its use. Furthermore, the drawings are schematic, and ratios of dimensions and the like do not necessarily match actual ones.

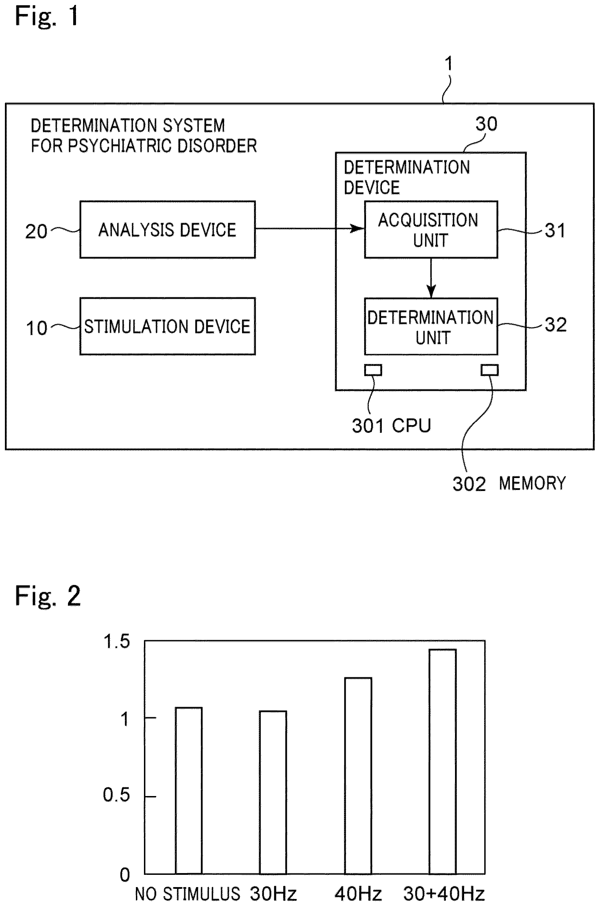

A determination system for a psychiatric disorder 1 according to an embodiment of the present disclosure includes, as an example, as illustrated in Fig. 1, a stimulation device 10, an analysis device 20, and a determination device 30. In the determination system 1, the analysis device 20 is configured to analyze a heart rate variability index (HRV) of a healthy person and a user under an environment no sound stimulus and under an environment where the stimulation device 10 gives a sound stimulus, and the determination device 30 is configured to determine a possibility that the user has a psychiatric disorder based on the analyzed heart rate variability index.

The stimulation device 10, the analysis device 20, and the determination device 30 may all be positioned at the same place or may each be positioned at different places. For example, the determination system 1 may be configured that the stimulation device 10 is built-in a smart watch and carried by the user, and the analysis device 20 and the determination device 30 are positioned in an external device different from the smart watch (for example, a server). As a result, for example, the determination system can correspond to the following embodiments.
· A patient or a medical examinee is subjected to medical treatment by a doctor remotely. In this case, for example, a pulse wave is acquired by photographing a face or a finger with a camera of a personal computer or a smartphone at home. A sound stimulus is given by using a microphone and a calling feature of a smartphone or a microphone and a speaker of a personal computer. A determination result from the determination device 30 can be diagnosis auxiliary data of the doctor.
· The determination system is used for stress checks or health examinations conducted at workplace, school, etc. In this case, a pulse wave is acquired by using a camera of a personal computer or a smartphone at a workplace, a school, etc., or an electrocardiogram is acquired by electrocardiographic measurement. A sound stimulus is given by using a microphone and a calling feature of a smartphone or a microphone and a speaker of a personal computer. In addition, for example, a pulse wave is acquired by using an earphone, glasses, or a ring (smart ring) in which a pulse wave sensor is embedded. A sound stimulus is given by using a microphone and a calling feature of an earphone or a smartphone or a microphone and a speaker of a personal computer.
· The determination system is mounted on a smartphone, a smartwatch, or the like, and is used by the user to check a state of his/her psychiatric disorder periodically (for example, every day) with the determination system. As a result, the user can not only manage a daily physical condition by himself/herself, but also visually recognize effects of receiving various treatments by himself/herself using the determination system.
· The determination system is applied to biofeedback, and the user uses a determination index as an indicator and controls the state of his/her psychiatric disorder.
· The determination system is used for biofeedback of digital medical treatments (cognitive behavioral therapy, music therapy, and the like) performed on a smartphone. The "biofeedback" means that information about unconscious processes (heart rate, blood pressure, muscle tension, etc.) is measured using the measuring device, and the information is presented in a form that allows the user to be aware of the information. By training, the patient can understand why such changes in physical function occur and learn how to control the changes in physical function, resulting in being capable of relief of symptoms such as pain, stress, insomnia, and headache.

The stimulation device 10 is configured to give a sound stimulus to the healthy person or the user, and to place the healthy person or the user under an "sound stimulus environment". The "sound stimulus environment" includes a first sound stimulus environment and a second sound stimulus environment different from the first sound stimulus environment. Under the first sound stimulus environment, no sound stimulus is given or a sound stimulus is given. Under the second sound stimulus environment, a sound stimulus is given. The sound stimulus given under the second sound stimulus environment includes a sound stimulus of a first frequency and a sound stimulus of a second frequency higher than the first frequency. The user is a person who is determined by the determination device 30 whether the person has a psychiatric disorder, and the healthy person is a person who does not suffer from a psychiatric disorder.

The analysis device 20 is configured to acquire and analyze the heart rate variability index of the healthy person or the user under the sound stimulus environment. The analysis device 20 is connected to the determination device 30 in a wireless or wired manner, for example. The heart rate variability index analyzed by the analysis device 20 is output to the determination device 30 by communication. Various heart rate variability indexes are well known (Front Public Health. 2017; 5: 258). The heart rate variability index includes, for example, low frequency (LF), high frequency (HF), LF/HF, pNN 50 (percent of heart rate with which a difference between consecutive adjacent RR intervals (interval from QRS wave to next QRS wave) greater than 50 ms).

In the present embodiment, the heart rate variability index is acquired and analyzed by a photoplethysmography (PPG) sensor (optical heart rate sensor) or an electrocardiogram (ECG) sensor as an example. In other words, the heart rate variability index is analyzed based on a pulse wave or an electrocardiogram. The PPG sensor or the ECG sensor may be included in the analysis device 20, or the stimulation device 10, or the determination device 30.

The determination device 30 includes an acquisition unit 31 and a determination unit 32. In the present embodiment, the determination device 30 includes a central processing unit (CPU) 301 that makes calculation, and a memory 302 configured to store a program necessary for the calculation and a calculation result. The acquisition unit 31 and the determination unit 32 each are achieved, for example, by the CPU 301 executing a predetermined program.

The acquisition unit 31 is configured to acquire a first reference value, a second reference value, a first comparison value, and a second comparison value. In the present embodiment, the acquisition unit 31 is configured to acquire from the analysis device 20 a third reference value, a fourth reference value, a third comparison value, and a fourth comparison value in addition to the first reference value, the second reference value, the first comparison value, and the second comparison value. The first reference value, the second reference value, the third reference value, the fourth reference value, the first comparison value, the second comparison value, the third comparison value, and the fourth comparison value are each, for example, a value calculated from at least one of a plurality of indexes including a high-frequency component of the heart rate variability index and a low-frequency component of the heart rate variability index, specifically, LF/HF that is a ratio of the low-frequency component to the high-frequency component.

The first reference value, the second reference value, the third reference value, the fourth reference value, the first comparison value, the second comparison value, the third comparison value, and the fourth comparison value are, for example, a heart rate variability index of the healthy person or the user in the following sound stimulus environments.
· First reference value: The heart rate variability index of the healthy person under the first sound stimulus environment. For example, the heart rate variability index of the healthy person to whom no sound stimulus is given or the healthy person to whom a sound stimulus of 30 Hz is given.
· Second reference value: The heart rate variability index of the healthy person under the second sound stimulus environment; the heart rate variability index of the healthy person to whom the sound stimulus of the first frequency or the second frequency is given. For example, the heart rate variability index of the healthy person to whom a sound stimulus of 30 Hz or 40 Hz is given. A heart rate variability index of the healthy person to whom the sound stimulus of the first frequency is given in a case where the third reference value is used. For example, the heart rate variability index of the healthy person to whom the sound stimulus of 30 Hz is given.
· Third reference value: The heart rate variability index of the healthy person under a third sound stimulus environment different from the first and second sound stimulus environments; the heart rate variability index of the healthy person to whom the sound stimulus of the second frequency is given. For example, the heart rate variability index of the healthy person to whom a sound stimulus of 40 Hz is given.
· Fourth reference value: The heart rate variability index of the healthy person under a fourth sound stimulus environment different from the first to third sound stimulus environments; the heart rate variability index of the healthy person to whom the sound stimulus of the first frequency and the sound stimulus of the second frequency are given simultaneously. For example, the heart rate variability index of the healthy person to whom the sound stimulus of 30 Hz and the sound stimulus of 40 Hz are given simultaneously.
· First comparison value: The heart rate variability index of the user under the first sound stimulus environment. For example, the heart rate variability index of the user to whom no sound stimulus is given or the user to whom the sound stimulus of 30 Hz is given.
· Second comparison value: The heart rate variability index of the user under the second sound stimulus environment; the heart rate variability index of the user to whom the sound stimulus of the first frequency or the second frequency is given. For example, the heart rate variability index of the user to whom the sound stimulus of 30 Hz or 40 Hz is given. The heart rate variability index of the user to whom the sound stimulus of the first frequency is given in a case where the third comparison value is used. For example, the heart rate variability index of the user to whom the sound stimulus of 30 Hz is given.
· Third comparison value: The heart rate variability index of the user under the third sound stimulus environment; the heart rate variability index of the user to whom the sound stimulus of the second frequency is given. For example, the heart rate variability index of the user to whom the sound stimulus of 40 Hz is given.
· Fourth comparison value: The heart rate variability index of the user under the fourth sound stimulus environment; the heart rate variability index of the user to whom the sound stimulus of the first frequency and the sound stimulus of the second frequency are given simultaneously. For example, the heart rate variability index of the healthy person to whom the sound stimulus of 30 Hz and the sound stimulus of 40 Hz are given simultaneously.

The sound stimulus environments each are not limited to the environment where no sound stimulus is given and the environment where one or both of the sound stimulus of 30 Hz and the sound stimulus of 40 Hz are given, and may be an environment where the mental state of a subject including the user and the healthy person can be determined. The first sound stimulus environment is not limited to an environment where no sound stimulus is given, and may be an environment where a sound stimulus of a predetermined frequency is given. The second to fourth sound stimulus environments each are preferably an environment where sound stimuli of 20 to 50 Hz are given, more preferably an environment where sound stimuli of 25 to 45 Hz are given, further preferably an environment where sound stimuli of 27 to 43 Hz are given, particularly preferably an environment where sound stimuli of 30 to 40 Hz are given, and most preferably an environment where sound stimuli of 30 Hz or 40 Hz are given. Further, these sound stimuli may be used in combination. For example, the sound stimuli of 30 Hz and 40 Hz may be given simultaneously, as in the fourth reference value and the fourth comparison value. A sound pressure in a case where the sound stimulus of the specific frequency is given may be any pressure of sound audible to the subject, and may be any sound pressure that enables a determination of the mental condition of the subject. In addition, these sound stimuli may be, for example, either a click sound or a continuous sound, and are not particularly limited.

For example, the acquisition unit 31 may be configured to acquire, as each reference value, the heart rate variability index of the healthy person measured each time when the determination device 30 is used, or may acquire, as each reference value, the heart rate variability index of the healthy person measured and stored at the time of using the determination device 30 in the past. The acquisition unit 31 may be configured to acquire each reference value by selecting from among reference values measured and accumulated each time when the determination device 30 is used.

The determination unit 32 is configured to execute comparison processing among the first reference value, the second reference value, the first comparison value, and the second comparison value to determine a possibility that the user has a psychiatric disorder. Examples of the determinable psychiatric disorder include states having symptoms such as depressive symptoms, manic symptoms, anxiety symptoms, and hallucinations and delusions. Examples of depressive symptoms include depressive episodes of a major depressive disorder (MDD) and a bipolar disorder. An example of the manic symptoms is a manic episode of the bipolar disorder. An example of the anxiety symptoms is an anxiety disorder (generalized anxiety disorder, a social anxiety disorder, etc.). An example of the hallucinations and delusions is schizophrenia. Further, examples of the determinable psychiatric disorder include a mood disorder or schizophrenia. The mood disorder includes depression or a bipolar disorder, and the bipolar disorder includes depressive episodes.

A possibility that the user has a psychiatric disorder is determined, for example, by the following comparison processing.
· Comparing a size of the first reference value and a size of the first comparison value.
· Comparing a size of the second reference value and a size of the second comparison value.
· Comparing a ratio of change of the second reference value to the first reference value and a ratio of change of the second comparison value to the first comparison value.

In the present embodiment, the determination unit 32 is configured to determine whether the user has a psychiatric disorder using the third reference value, the fourth reference value, the third comparison value, and the fourth comparison value in addition to the first reference value, the second reference value, the first comparison value, and the second comparison value. In this case, whether the user has a psychiatric disorder is determined, for example, by the following comparison processing in addition to the above-described comparison processing method.
· Comparing a size of the third reference value and a size of the third comparison value.
· Comparing a ratio of change of the third reference value to the first reference value and a ratio of change of the third comparison value to the first comparison value.
· Comparing a size of the fourth reference value and a size of the fourth comparison value.
· Comparing a ratio of change of the fourth reference value to the first reference value and a ratio of change of the fourth comparison value to the first comparison value.
· Comparing the size of the third reference value and the size of the third comparison value, and comparing the size of the fourth reference value and the size of the fourth comparison value.
· Comparing the ratio of change of the third reference value to the first reference value and the ratio of change of the third comparison value to the first comparison value, and comparing the ratio of change of the fourth reference value to the first reference value and the ratio of change of the fourth comparison value to the first comparison value.

For example, under the environments of "no sound stimulus", "giving the sound stimulus of 30 Hz", "giving the sound stimulus of 40 Hz", and "giving the sound stimuli of 30 Hz + 40 Hz", LF/HF of "patients with individual psychiatric disorders (bipolar I disorder and schizophrenia)" and LF/HF of "healthy persons" show tendencies as illustrated in Figs. 2 to 4. In Figs. 2 to 4, the first reference value, the second reference value, the third reference value, the fourth reference value, the first comparison value, the second comparison value, the third comparison value, and the fourth comparison value are heart rate variability indexes described below.
· First reference value: The heart rate variability index of the healthy person to whom no sound stimulus is given.
· Second reference value: The heart rate variability index of the healthy person to whom the sound stimulus of 30 Hz is given.
· Third reference value: The heart rate variability index of the healthy person to whom the sound stimulus of 40 Hz is given.
· Fourth reference value: The heart rate variability index of the healthy person to whom the sound stimulus of 30 Hz and the sound stimulus of 40 Hz are given simultaneously.
· First comparison value: The heart rate variability index of the user to whom no sound stimulus is given.
· Second comparison value: The heart rate variability index of the user to whom the sound stimulus of 30 Hz is given.
· Third comparison value: The heart rate variability index of the user to whom the sound stimulus of 40 Hz is given.
· Fourth comparison value: The heart rate variability index of the healthy person to whom the sound stimulus of 30 Hz and the sound stimulus of 40 Hz are given simultaneously.

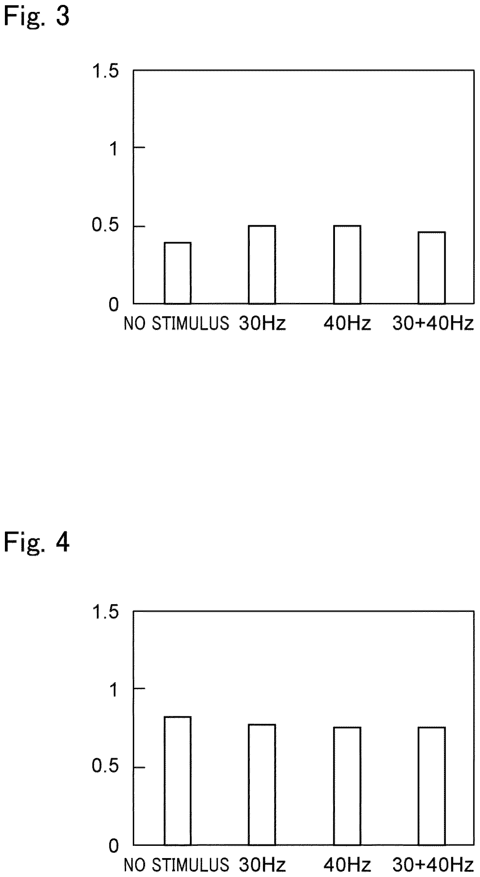

Fig. 2 illustrates LF/HF of the "healthy persons". Fig. 3 illustrates LF/HF of "patients with bipolar I disorder". Fig. 4 illustrates LF/HF of "patients with schizophrenia". Figs. 2 to 4 each illustrate, as an example, the LF/HF calculated under the following conditions.
· Pulse wave data was acquired from patients and healthy persons (15 persons) using the PPG sensor under the environments of "no sound stimulus", "giving sound of 30 Hz", "giving sound of 40 Hz", and "giving sounds of 30 Hz + 40 Hz". The patients, which are in a hospital or visit a hospital, have psychiatric disorders (15 patients with schizophrenia, and 11 patients with the bipolar I disorder). In Figs. 3 and 4, LF/HF of each patient is an average value of LF/HF of each of all patients from whom pulse wave data is acquired. For example, LF/HF of a depressed patient is the average value of LF/HF of all depressed patients from whom pulse wave data is acquired.
· Each sound stimulus was set as a stimulus that was given to the healthy person or the user for 150 sets (150 seconds in total) in which one set includes a click sound "that is not given for 500 msec after giving for 500 msec". The click sound has a frequency corresponding to each sound stimulus (that is, 30 Hz, 40 Hz, or 30 Hz + 40 Hz) and a sound pressure of 70 dB.
· From the acquired pulse wave data, the analysis device 20 analyzed various heart rate variability parameters (LF, HF, LF/HF, pNN 50, etc.). As for HF and LF, power spectrum analysis was performed on frequency components of a periodic variation of the pulse wave in the acquired pulse wave data (RR interval), and the frequency components were classified into three frequency regions including VLF: a low-frequency component (0 to 0.05 Hz), LF: an intermediate-frequency component (0.05 to 0.20 Hz), and HF: a high-frequency component (0.20 to 0.35 Hz). LF/HF was calculated from the classified LF and HF.

### (Patient with bipolar I disorder)

As illustrated in Figs. 2 and 3, the "patients with bipolar I disorder" tend to have LF/HF lower than the "healthy person" under any environments of "no sound stimulus", "giving the sound stimulus of 30 Hz", "giving the sound stimulus of 40 Hz", and "giving the sound stimuli of 30 Hz + 40 Hz". Under the environments of "giving the sound stimulus of 40 Hz" and "giving the sound stimuli of 30 Hz + 40 Hz", the "healthy persons" tend to have higher LF/HF than LF/HF under the environment of "no sound stimulus", whereas the "patients with bipolar I disorder" tend to have LF/HF approximately equal to LF/HF under the environment of "no sound stimulus". As illustrated in Figs. 2 and 3, the "patients with bipolar I disorder" have LF/HF lower than the "healthy persons" under any environments of "no sound stimulus", "giving the sound stimulus of 30 Hz", "giving the sound stimulus of 40 Hz", and "giving the sound stimuli of 30 Hz + 40 Hz".

For example, with reference to Figs. 2 and 3, in a case where any one, any two or more, or all of the following conditions is/are satisfied, a determination is made that "the user may have the bipolar I disorder".
· The first comparison value is smaller than the first reference value.
· The second comparison value is smaller than the second reference value.
· The third comparison value is smaller than the third reference value.
· The fourth comparison value is smaller than the fourth reference value.
· The ratio of change of the third comparison value to the first comparison value is smaller than the ratio of change of the third reference value to the first reference value.
· The ratio of change of the fourth comparison value to the first comparison value is smaller than the ratio of change of the fourth reference value to the first reference value.
· The ratio of change of the third comparison value to the second comparison value is smaller than the ratio of change of the third reference value to the second reference value.
· The ratio of change of the fourth comparison value to the second comparison value is smaller than the ratio of change of the fourth reference value to the second reference value.

In the determination, it is considered that a possibility that the user has the bipolar I disorder can be more accurately determined by enhancing an importance of a result of the next comparison processing.
· "Comparison between the ratio of change of the third reference value to the first reference value and the ratio of change of the third comparison value to the first comparison value".
· "Comparison between the ratio of change of the fourth reference value to the first reference value and the ratio of change of the fourth comparison value to the first comparison value".
· "Comparison between each reference value and each comparison value".

### (Patient with schizophrenia)

As illustrated in Figs. 2 and 4, no large difference between the "patients with schizophrenia" and the healthy persons is observed under the environments of "no sound stimulus" and "giving the sound stimulus of 30 Hz", but LF/HF of the "patients with schizophrenia" tend to be lower than LF/HF of the "healthy persons" under the environments of "giving the sound stimulus of 40 Hz" and "giving the sound stimuli of 30 Hz + 40 Hz". Under the environments of "giving the sound stimulus of 40 Hz" and "giving the sound stimuli of 30 Hz + 40 Hz", the "healthy persons" tend to have higher LF/HF than LF/HF under the environment of "no sound stimulus", whereas the "patients with schizophrenia" tend to have LF/HF approximately equal to LF/HF under the environment of "no sound stimulus". As illustrated in Figs. 3 and 4, the "patients with schizophrenia" have higher LF/HF than the "patients with bipolar I disorder" under the environment of "no sound stimulus".

For example, with reference to Figs. 2 to 4, in a case where any one, any two or more, or all of the following conditions is/are satisfied, a determination is made that "the user may have schizophrenia".
· There is no large difference between the first comparison value and the first reference value.
· There is no large difference between the second comparison value and the second reference value.
· The third comparison value is smaller than the third reference value.
· The fourth comparison value is smaller than the fourth reference value.
· The ratio of change of the third comparison value to the first comparison value is smaller than the ratio of change of the third reference value to the first reference value.
· The ratio of change of the fourth comparison value to the first comparison value is smaller than the ratio of change of the fourth reference value to the first reference value.
· A ratio of change of the third comparison value to the second comparison value is smaller than a ratio of change of the third reference value to the second reference value.
· A ratio of change of the fourth comparison value to the second comparison value is smaller than a ratio of change of the fourth reference value to the second reference value.

In the determination, it is considered that a possibility that the user has schizophrenia can be determined more accurately by enhancing an importance of a result of the next comparison processing.
· "Comparison between the ratio of change of the third reference value to the first reference value and the ratio of change of the third comparison value to the first comparison value"
· "Comparison between the ratio of change of the fourth reference value to the first reference value and the ratio of change of the fourth comparison value to the first comparison value"
• "Comparison between the first reference value and first comparison value"
· "Comparison between the second reference value and the second comparison value"

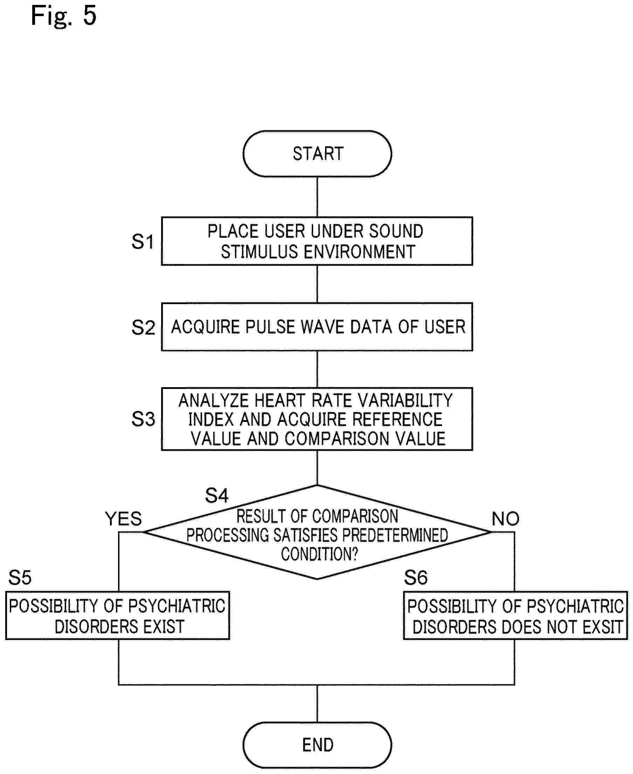

Next, the determination method for a psychiatric disorder will be described with reference to Fig. 5. Here, as an example, a case where the comparison processing is executed among the first reference value, the first comparison value, the second reference value, and the second comparison value, and a possibility of a psychiatric disorder is determined will be described.

As illustrated in Fig. 5, the user is placed under the first sound stimulus environment and the second sound stimulus environment by the stimulation device 10 (step S1), and the pulse wave data of the user is acquired (step S2).

When the pulse wave data of the user is acquired, the analysis device 20 analyzes the heart rate variability index from the acquired pulse wave data, and the acquisition unit 31 of the determination device 30 acquires the first reference value, the second reference value, the first comparison value, and the second comparison value which values are the analyzed heart rate variability indexes (step S3).

When the first reference value, the second reference value, the first comparison value, and the second comparison value are acquired, the determination unit 32 of the determination device 30 executes comparison processing among the first reference value, the first comparison value, the second reference value, and the second comparison value, and determines whether a predetermined condition is satisfied (step S4). The predetermined condition is individually set depending on a psychiatric disorder that is a target to be determined. For example, the predetermined condition is satisfied in a case where any one, any two or all of three conditions including "the first comparison value is smaller than the first reference value", "the second comparison value is smaller than the second reference value", and "the ratio of change of the second comparison value to the first comparison value is small" is/are satisfied.

When the determination is made that the predetermined condition is satisfied, the determination unit 32 determines that "a possibility of a psychiatric disorder exists" (step 5). On the other hand, when the determination is made that the predetermined condition is not satisfied, the determination unit 32 determines that "a possibility of a psychiatric disorder does not exist" (step S6). When the determination unit 32 determines a possibility that the user has a psychiatric disorder, the determination method for a psychiatric disorder ends.

The determination method for a psychiatric disorder of the present disclosure can exhibit the following advantageous effects.

In the determination method for a psychiatric disorder of present disclosure, the first reference value, the second reference value, the first comparison value, and the second comparison value are acquired. The first reference value is the heart rate variability index of the healthy person under the first sound stimulus environment, and the second reference value is the heart rate variability index of the healthy person under the second sound stimulus environment different from the first sound stimulus environment. The first comparison value is the heart rate variability index of the user under the first sound stimulus environment, and the second comparison value is the heart rate variability index of the user under the second sound stimulus environment. The comparison processing is executed among the first reference value, the second reference value, the first comparison value, and the second comparison value, and a possibility that the user has a psychiatric disorder. Such a configuration makes it possible to achieve the determination method for a psychiatric disorder that can be used more easily. In the determination method for a psychiatric disorder of the present disclosure, it is not necessary to show a video at the time of use, and thus the determination method has less strain on the user, and can be used not only by a subject who has no visual impairment but also by a subject who has visual impairment. The determination method for a psychiatric disorder of the present disclosure is not affected by at least a preference of the user as to a video, and thus a restriction on the determination method can be reduced.

As described above, the determination method for a psychiatric disorder of the present disclosure can be used for a determination as to various psychiatric disorders, and for example, can determine a possibility of bipolar I disorder and schizophrenia. In addition, since the determination method determines a possibility of a psychiatric disorders based on the heart rate variability index of the user under the sound stimulus environment, a strain on the user in the determination is less.

The program of the present disclosure can cause a computer to execute the determination method for a psychiatric disorder that can be used more easily.

The determination device 30 can achieve a determination device for a psychiatric disorder that can be used more easily.

The determination system for a psychiatric disorder 1 can achieve by the determination device 30 determination system that can be used more easily.

The determination method for a psychiatric disorder, the program, the determination device for a psychiatric disorder 30, and the determination system for a psychiatric disorder 1 may optionally adopt any one or a plurality of configurations described below. That is, any one or more configurations of the following plurality of configurations may be optionally deleted in a case of being included in the embodiment, and can be optionally added in a case of not being included in the embodiment. The adoption of such a configuration enables a more reliable determination as to a possibility that the user has a psychiatric disorder.

The first reference value, the second reference value, the first comparison value, and the second comparison value each are a value calculated based on at least one of a plurality of indexes including a high-frequency component of the heart rate variability index and a low-frequency component of the heart rate variability index.

The first reference value, the second reference value, the first comparison value, and the second comparison value each are LF/HF that is the ratio of the low-frequency component to the high-frequency component.

The heart rate variability index is analyzed from based on a pulse wave or an electrocardiogram.

A possibility that the user has a psychiatric disorder is determined by comparing the first reference value and the first comparison value.

A possibility that the user has a psychiatric disorder is determined by comparing the second reference value and the second comparison value.

A possibility that the user has a psychiatric disorder is determined by comparing the ratio of change of the second reference value to the first reference value and the ratio of change of the second comparison value to the first comparison value.

Under the first sound stimulus environment, no sound stimulus is given or a sound stimulus is given, and under the second sound stimulus environment, a sound stimulus is given.

A sound stimulus given under the second sound stimulus environment includes the sound stimulus of the first frequency and the sound stimulus of the second frequency higher than the first frequency. The second reference value is the heart rate variability index of the healthy person to whom the sound stimulus of the first frequency is given, and the second comparison value is the heart rate variability index of the user to whom the sound stimulus of the first frequency is given. In addition to the first reference value, the second reference value, the first comparison value, and the second comparison value, the third reference value that is the heart rate variability index of the healthy person to whom the sound stimulus of the second frequency is given and the third comparison value that is the heart rate variability index of the user to whom the sound stimulus of the second frequency is given are acquired. The comparison processing is executed among the first reference value, the second reference value, the third reference value, the first comparison value, the second comparison value, and the third comparison value to determine a possibility that the user has a psychiatric disorder.

A possibility that the user has a psychiatric disorder is determined by comparing the third reference value and the third comparison value.

A possibility that the user has a psychiatric disorder is determined by comparing the ratio of change of the third reference value to the first reference value and the ratio of change of the third comparison value to the first comparison value.

A sound stimulus given under the second sound stimulus environment includes the sound stimulus of the first frequency and the sound stimulus of the second frequency higher than the first frequency. The second reference value is the heart rate variability index of the healthy person to whom the sound stimulus of the first frequency is given, and the second comparison value is the heart rate variability index of the user to whom the sound stimulus of the first frequency is given. In addition to the first reference value, the second reference value, the first comparison value, and the second comparison value, the fourth reference value that is the heart rate variability index of the healthy person to whom the sound stimulus of the first frequency and the sound stimulus of the second frequency are given simultaneously, and the fourth comparison value that is the heart rate variability index of the user to whom the sound stimulus of the first frequency and the sound stimulus of the second frequency are given simultaneously are acquired. The comparison processing is executed among the first reference value, the second reference value, the fourth reference value, the first comparison value, the second comparison value, and the fourth comparison value to determine a possibility that the user has a psychiatric disorder.

A possibility that the user has a psychiatric disorder is determined by comparing the fourth reference value and the fourth comparison value.

A possibility that the user has a psychiatric disorder is determined by comparing the ratio of change of the fourth reference value to the first reference value and the ratio of change of the fourth comparison value to the first comparison value.

A sound stimulus given under the second sound stimulus environment includes the sound stimulus of the first frequency and the sound stimulus of the second frequency higher than the first frequency. The second reference value is the heart rate variability index of the healthy person to whom the sound stimulus of the first frequency is given, and the second comparison value is the heart rate variability index of the user to whom the sound stimulus of the first frequency is given. In addition to the first reference value, the second reference value, the first comparison value, and the second comparison value, the third reference value, the third comparison value, the fourth reference value, and the fourth comparison value are acquired. The third reference value is the heart rate variability index of the healthy person to whom the sound stimulus of the second frequency is given. The third comparison value is the heart rate variability index of the user to whom the sound stimulus of the second frequency is given. The fourth reference value is the heart rate variability index of the healthy person to whom the sound stimulus of the first frequency and the sound stimulus of the second frequency are simultaneously given. The fourth comparison value is the heart rate variability index of the user to whom the sound stimulus of the first frequency and the sound stimulus of the second frequency are simultaneously given. The comparison processing is executed among the first reference value, the second reference value, the third reference value, the fourth reference value, the first comparison value, the second comparison value, the third comparison value, and the fourth comparison value to determine a possibility that the user has a psychiatric disorder.

A possibility that the user has a psychiatric disorder is determined by comparing the third reference value and the third comparison value, and comparing the fourth reference value and the fourth comparison value.

A possibility that the user has a psychiatric disorder is determined by comparing the ratio of change of the third reference value to the first reference value and the ratio of change of the third comparison value to the first comparison value, and comparing the ratio of change of the fourth reference value to the first reference value and the ratio of change of the fourth comparison value to the first comparison value.

A sound stimulus given under the second sound stimulus environment includes the sound stimulus of the first frequency and the sound stimulus of the second frequency higher than the first frequency. The second reference value is the heart rate variability index of the healthy person to whom the sound stimulus of the second frequency is given, and the second comparison value is the heart rate variability index of the user to whom the sound stimulus of the second frequency is given.

The psychiatric disorder is a mood disorder or schizophrenia.

The mood disorder is depression or bipolar disorder.

The bipolar disorder is a depressive episode.

The determination method for a psychiatric disorder, the determination device 30, and the determination system for a psychiatric disorder 1 may be configured as follows.

The determination as to a possibility that the user has a psychiatric disorder may be determined by a statistical regression model. That is, the "comparison processing" executed among the first reference value, the second reference value, the third reference value, the fourth reference value, the first comparison value, the second comparison value, the third comparison value, and the fourth comparison value includes the statistical regression model such as logistic regression analysis. The statistical regression model is constructed by mechanically selecting (for example, selecting up to three variables using a mechanical variable increasing method) a variable from various variable sets (LF, HF, LF/HF, etc.) obtained based on a pulse wave data analysis of the patient with a psychiatric disorder (for example, depression or bipolar II disorder). As for selecting a variable, for example, a variable selection method described in "Loann David Denis Desboulets et al: A Review on Variable Selection in Regression Analysis. Econometrics 6, 45 (2018)" may be used.

Each of the determination method for a psychiatric disorder and the determination device 30 may be configured to determine a possibility that the user has a psychiatric disorder by executing the comparison processing using at least the first reference value, the second reference value, the first comparison value, and the second comparison value. For example, the comparison processing may be executed using the third reference value, the fourth reference value, the third comparison value, and the fourth comparison value, or may be executed using a fifth reference value different from the first to fourth reference values and a fifth comparison value different from the first to fourth comparison values, in addition to the first to fourth reference values and the first to fourth comparison values.

Each reference value and each comparison value are not limited to LF/HF, and may be other parameters.

The heart rate variability index may be obtained from pulse wave data. The pulse wave data may be acquired by, for example, the following method.
· Using a microwave radar.
· Using a pulse oximeter. The pulse wave data is acquired from arterial oxygen saturation (SpO2).
· Using a thermography. The pulse wave data is acquired from a change in temperature of a face or a neck.
· Using a smart watch capable of measuring temperature of a body surface. The pulse wave data is acquired from a change in temperature of a body surface.
· Using an earphone capable of measuring temperature of an inside of an ear. The pulse wave data is acquired using a vital sensing sensor or the like or from a change in temperature of the inside of the ear.

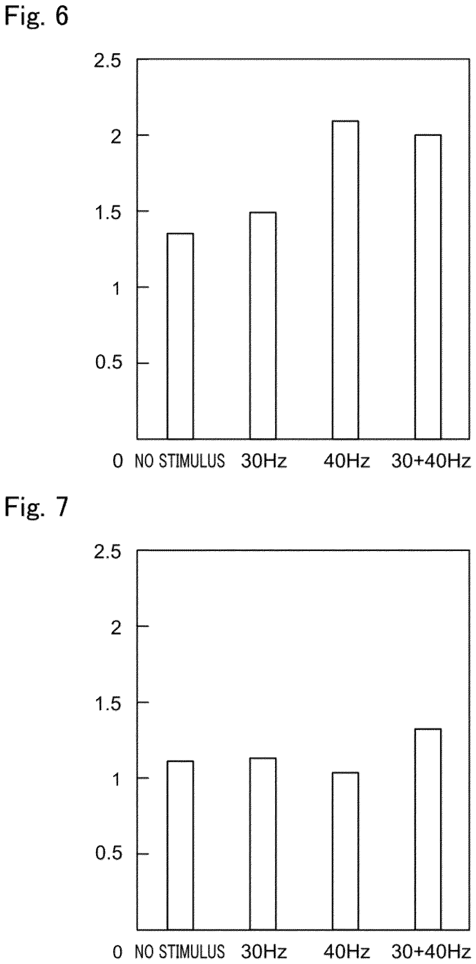

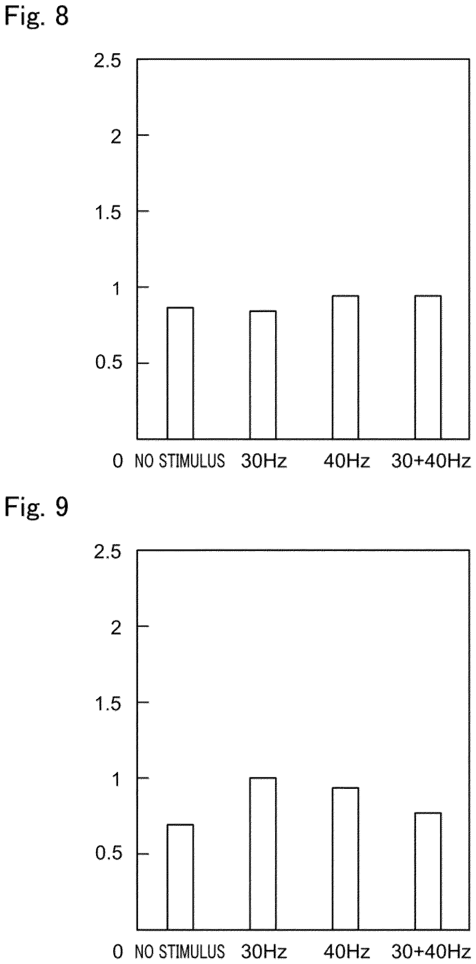

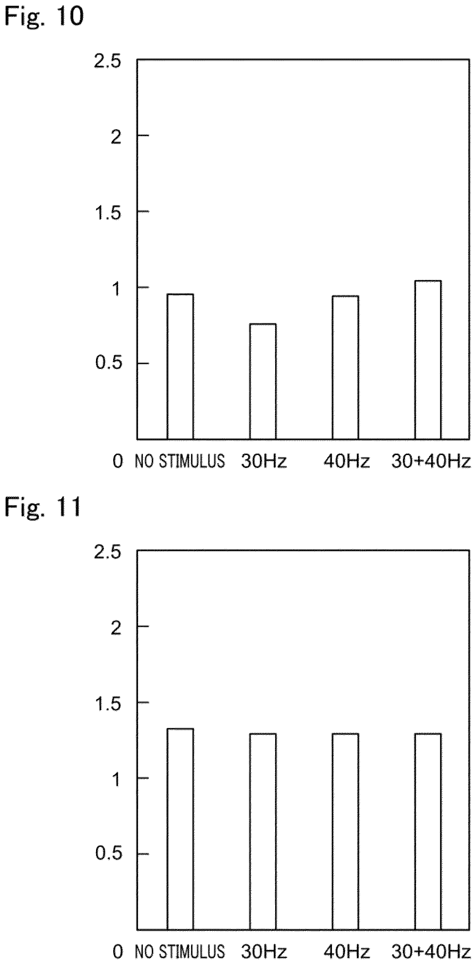

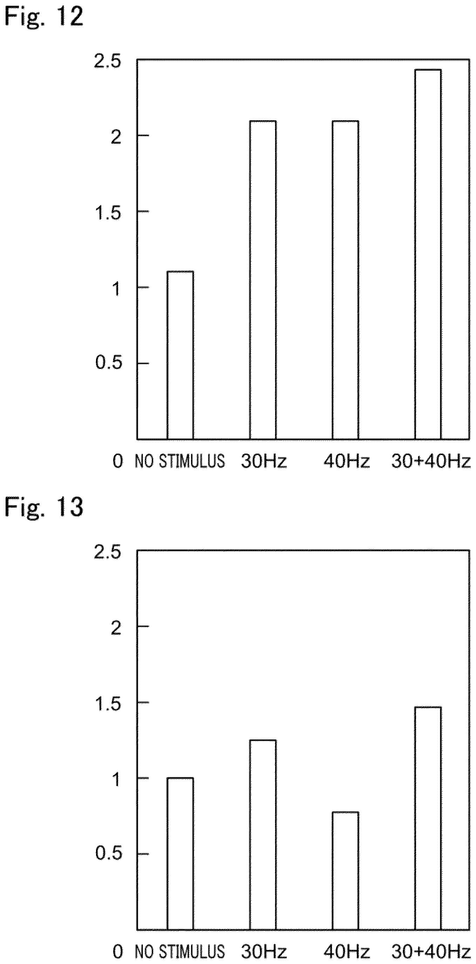

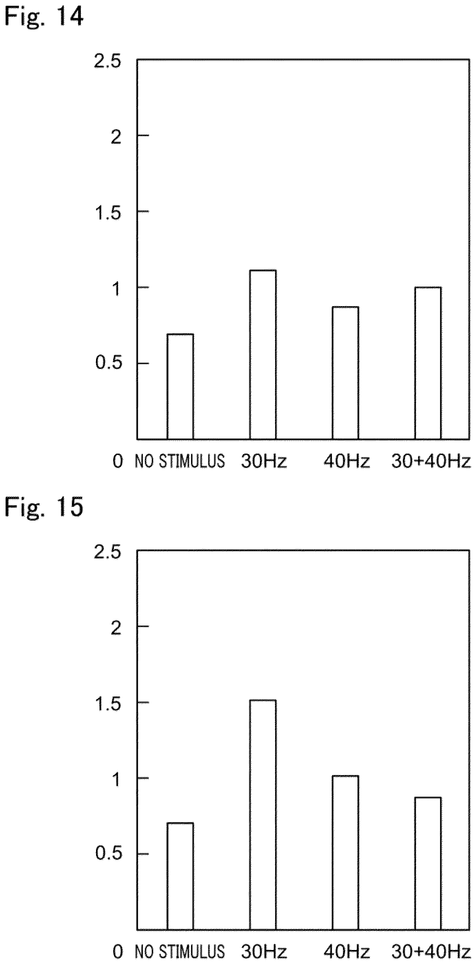

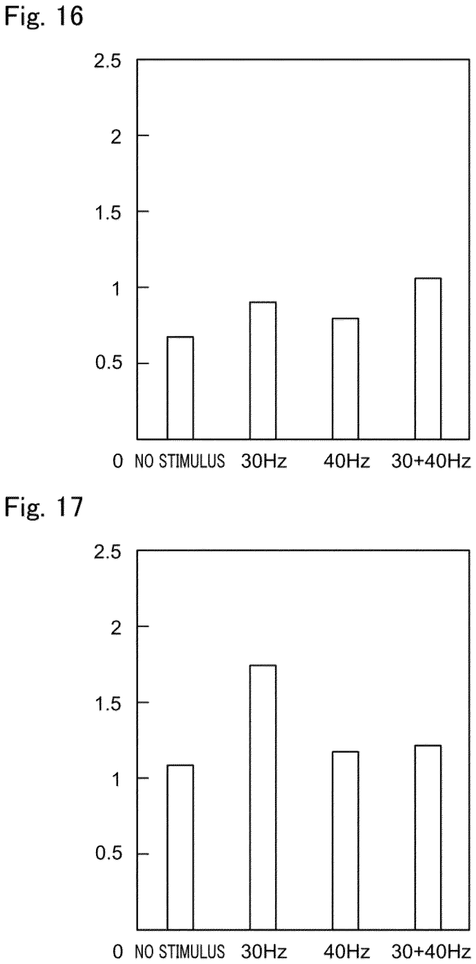

Various heart rate variability parameters (LF, HF, LF/HF, pNN 50, etc.) may be analyzed by power spectrum analysis. Further, in order to observe a temporal change in various heart rate variability parameters, evaluation may be performed by a moving average method. As an example, Figs. 6 to 17 illustrate values for 60 seconds or 150 seconds immediately after each sound stimulus among values of LF/HF analyzed by shifting pulse wave data every 1 second for 60 seconds or 150 seconds under the following conditions. Figs. 6 and 12 illustrate average LF/HF of the "healthy persons". Figs. 7 and 13, show values of LF/HF of the "depression". Figs. 8 and 14 show LF/HF values of the "bipolar disorder (BP1 + BP2)". Figs. 9 and 15 show LF/HF values of the "bipolar I disorder (BP1)". Figs. 10 and 16 show LF/HF values of the "bipolar II disorder (BP2)". Figs. 11 and 17 show values of LF/HF of the "schizophrenia".
· The pulse wave data was acquired from the patients and the healthy persons (25 persons) using the PPG sensor under the environments of "no sound stimulus", "giving sound of 30 Hz", "giving sound of 40 Hz", and "giving sounds of 30 Hz + 40 Hz". The patients, which are in a hospital or visit a hospital, have psychiatric disorders (30 patients with depression, 11 patients with bipolar I disorder, 21 patients with bipolar II disorder, and 32 patients with schizophrenia).
· Each sound stimulus was set as a stimulus that was given to the healthy person or the user for 150 sets (150 seconds in total), in which one set includes a click sound "that is given for 500 msec and then after that is not given for 500 msec". The click sound has a frequency corresponding to each sound stimulus (that is, 30 Hz, 40 Hz, or 30 Hz + 40 Hz) and a sound pressure of 70 dB. The pulse wave data for 150 seconds (600 seconds in total) of each sound stimulus was subjected to the power spectrum analysis. LF/HF of the pulse wave data for 150 seconds immediately after each sound stimulus is shown in Figs. 6 to 11, and LF/HF of the pulse wave data for 60 seconds immediately after each sound stimulus is shown in Figs. 12 to 17. Note that the number of seconds of measurement is not limited to 150 seconds and 60 seconds, and any number of seconds may be selected. Further, a possibility of being a psychiatric disorder may be determined using, for example, either a measurement result of 150 seconds or a measurement result of 60 seconds, or may be determined using both the measurement result of 150 seconds and the measurement result of 60 seconds.
· From the acquired pulse wave data, the analysis device 20 analyzed various heart rate variability parameters (LF, HF, LF/HF, pNN 50, etc.). As for HF and LF, frequency components of a periodic variation of the pulse wave in the acquired pulse wave data (RR interval) were analyzed, and the frequency components were classified into three frequency regions including VLF: a low-frequency component (0 to 0.05 Hz), LF: an intermediate-frequency component (0.05 to 0.20 Hz), and HF: a high-frequency component (0.20 to 0.35 Hz). LF/HF was calculated from the classified LF and HF.

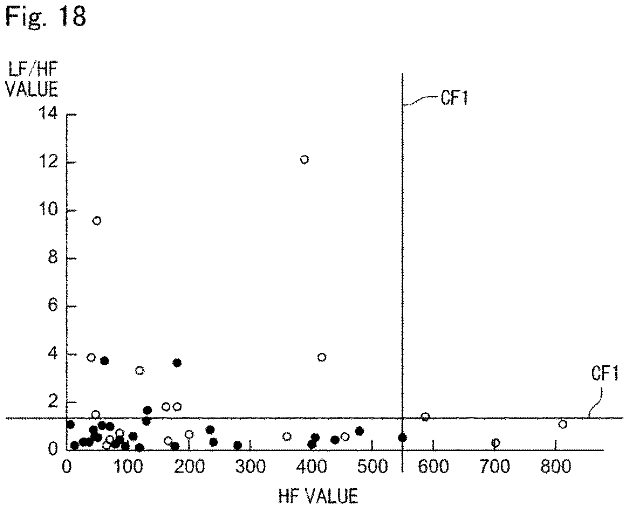

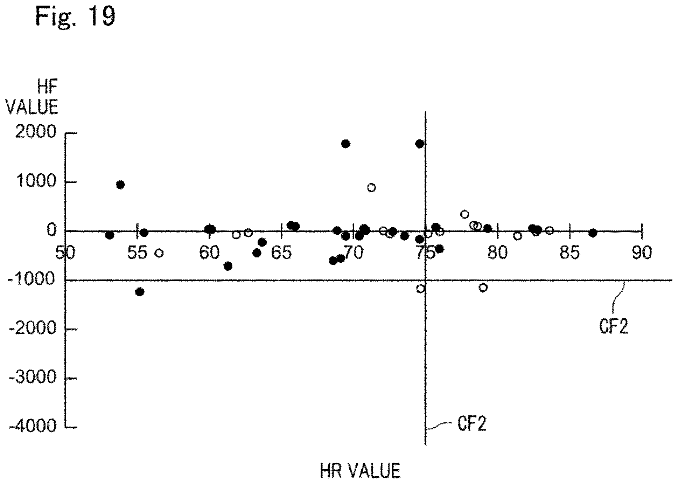

A possibility of being a psychiatric disorder is not limited to the case of the determination using only LF/HF, and may be determined using a combination of LF/HF and another index, or may be determined using a combination of a plurality of other indexes other than LF/HF. Fig. 18 shows an example of an analysis result using LF/HF and HF in combination, and Fig. 19 shows an example of an analysis result using HF and HR (heart rate: an average value of all heart rates in predetermined period (for example, 1 minute, 2 minutes, 3 minutes, 4 minutes, or 5 minutes)) in combination. As illustrated in Figs. 18 and 19, as the result of combining LF/HF and HF, or HF and HR, it becomes able to determine "depression" or the "bipolar disorder".

Fig. 18 illustrates an example of a case where, for example, a cut-off value CF1 is set to "HF: 550, LF/HF: 1.2" using HF in the case of "no sound stimulus" and LF/HF in the case of "giving sound of 40 Hz (60 seconds)" among the heart rate variability parameters of the "healthy person" and the patient with "depression" as indexes. In Fig. 18, an analysis result of the "healthy person" is indicated by a white circle, and an analysis result of the "patient with depression" is indicated by a black circle. In this case, a sensitivity is 80%, and a specificity is 70%.

Fig. 19 illustrates an example of a case where, for example, a cut-off value CF2 is set to "HR: 75, HF: -1000" using HR in the case of "giving sound of 40 Hz" and HF in the case of "giving sound of 40 Hz (30 seconds)" of the "healthy person" and the patient with "bipolar disorder" as indexes. In Fig. 19, an analysis result of the "healthy person" is indicated by a white circle, and an analysis result of "patient with bipolar disorder" is indicated by a black circle. In this case, a sensitivity is 75%, and a specificity is 76%.

The "cut-off value" is a boundary value that separates patients suffering from a specific disease and patients not suffering from the specific disease depending on the test result, and is also called a disease state discrimination value. The "sensitivity" indicates a rate of a positive determination among those having a disease, and the "specificity" indicates a rate of a negative determination among those having no disease.

Various embodiments of the present disclosure are described above in detail with reference to the drawings. Finally, various aspects of the present disclosure will be described.

A first aspect provides a determination method, including:
acquiring a first reference value, a second reference value, a first comparison value, and a second comparison value, the first reference value being a heart rate variability index of a healthy person under a first sound stimulus environment, the second reference value being a heart rate variability index of the healthy person under a second sound stimulus environment different from the first sound stimulus environment, the first comparison value being a heart rate variability index of a user under the first sound stimulus environment, the second comparison value being a heart rate variability index of the user under the second sound stimulus environment; and
executing comparison processing among the first reference value, the second reference value, the first comparison value, and the second comparison value to determine a possibility that the user has a psychiatric disorder.

A second aspect provides the determination method according to the first aspect, wherein
the first reference value, the second reference value, the first comparison value, and the second comparison value each are a value calculated based on at least one of a plurality of indexes including a high-frequency component of a heart rate variability index and a low-frequency component of a heart rate variability index.

A third aspect provides the determination method according to the second aspect, wherein
the first reference value, the second reference value, the first comparison value, and the second comparison value each are LF/HF that is a ratio of the low-frequency component to the high-frequency component.

A fourth aspect provides the determination method according to any one of the first to third aspects, wherein
the heart rate variability index is analyzed from a pulse wave or an electrocardiogram.

A fifth aspect provides the determination method according to any one of the first to fourth aspects, wherein
a possibility that the user has a psychiatric disorder is determined by comparing the first reference value with the first comparison value.

A sixth aspect provides the determination method according to any one of the first to fifth aspects, wherein
a possibility that the user has a psychiatric disorder is determined by comparing the second reference value with the second comparison value.

A seventh aspect provides the determination method according to any one of the first to sixth aspects, wherein
a possibility that the user has a psychiatric disorder is determined by comparing a rate of change of the second reference value to the first reference value and a rate of change of the second comparison value to the first comparison value.

An eighth aspect provides the determination method according to any one of the first to seventh aspects, wherein
under the first sound stimulus environment, no sound stimulus is given or a sound stimulus is given, and
under the second sound stimulus environment, a sound stimulus is given.

A ninth aspect provides the determination method according to the eighth aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given, and
the method comprises:
   acquiring a third reference value and a third comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, and
   executing comparison processing among the first reference value, the second reference value, the third reference value, the first comparison value, the second comparison value, and the third comparison value to determine a possibility that the user has a psychiatric disorder.

A tenth aspect provides the determination method according to the ninth aspect, wherein
a possibility that the user has a psychiatric disorder is determined by comparing the third reference value with the third comparison value.

An eleventh aspect provides the determination method according to the ninth or tenth aspect, wherein
a possibility that the user has a psychiatric disorder is determined by comparing a rate of change of the third reference value to the first reference value and a rate of change of the third comparison value to the first comparison value.

A twelfth aspect provides the determination method according to the eighth aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given, and
the method comprises:
acquiring a fourth reference value and a fourth comparison value in addition to the first reference value, the second reference value, the first comparison value, and the second comparison value, the fourth reference value being a heart rate variability index of the healthy person to whom the sound stimulus of the first frequency and the sound stimulus of the second frequency are given simultaneously, the fourth comparison value being a heart rate variability index of the user to whom the sound stimulus of the first frequency and the sound stimulus of the second frequency are given simultaneously, and
executing comparison processing among the first reference value, the second reference value, the fourth reference value, the first comparison value, the second comparison value, and the fourth comparison value to determine a possibility that the user has a psychiatric disorder.

A thirteenth aspect provides the determination method according to the twelfth aspect, wherein
a possibility that the user has a psychiatric disorder is determined by comparing the fourth reference value with the fourth comparison value.

A fourteenth aspect provides the determination method according to the twelfth or thirteenth aspect, wherein
a possibility that the user has a psychiatric disorder is determined by comparing a rate of change of the fourth reference value to the first reference value and a rate of change of the fourth comparison value to the first comparison value.

A fifteenth aspect provides the determination method according to the eighth aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given, and
the method comprises:
   acquiring a third reference value, a third comparison value, a fourth reference value, and a fourth comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, the fourth reference value being a heart rate variability index of the healthy person to whom the sound stimulus of the first frequency and the sound stimulus of the second frequency are given simultaneously, the fourth comparison value that being a heart rate variability index of the user to whom the sound stimulus of the first frequency and the sound stimulus of the second frequency are given simultaneously, and
   executing comparison processing among the first reference value, the second reference value, the third reference value, the fourth reference value, the first comparison value, the second comparison value, the third comparison value, and the fourth comparison value to determine a possibility that the user has a psychiatric disorder.

A sixteenth aspect provides the determination method according to the fifteenth aspect, wherein
a possibility that the user has a psychiatric disorder is determined by comparing the third reference value with the third comparison value and comparing the fourth reference value with the fourth comparison value.

A seventeenth aspect provides the determination method according to the fifteenth or sixteenth aspect, wherein
a possibility that the user has a psychiatric disorder is determined by comparing a rate of change of the third reference value to the first reference value and a rate of change of the third comparison value to the first comparison value, and comparing a rate of change of the fourth reference value to the first reference value and a rate of change of the fourth comparison value to the first comparison value.

An eighteenth aspect provides the determination method according to the eighth aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the second frequency is given, and
the second comparison value is a heart rate variability index of the user to whom the sound stimulus of the second frequency is given.

A nineteenth aspect provides the determination method according to any one of the first to eighteenth aspects, wherein
the psychiatric disorder is a state of depressive symptoms, manic symptoms, anxiety symptoms, or hallucinations and delusions.

A twentieth aspect provides the determination method according to any one of the first to eighteenth aspects, wherein
the psychiatric disorder is a mood disorder, an anxiety disorder, or schizophrenia.

A twenty-first aspect provides the determination method according to any one of the first to eighteenth aspects, wherein
the psychiatric disorder is a mood disorder or schizophrenia.

A twenty-second aspect provides the determination method according to the twenty-first aspect, wherein
the mood disorder is depression or a bipolar disorder.

A twenty-third aspect provides the determination method according to the twenty-second aspect, wherein
the bipolar disorder is a depressive episode.

A twenty-fourth aspect provides a program for causing a computer to execute the determination method according to any one of the first to twenty-third aspects.

A twenty-fifth aspect provides a determination device, including:
an acquisition unit configured to acquire a first reference value, a second reference value, a first comparison value, and a second comparison value, the first reference value being a heart rate variability index of a healthy person under a first sound stimulus environment, the second reference value being a heart rate variability index of the healthy person under a second sound stimulus environment, the first comparison value being a heart rate variability index of a user under the first sound stimulus environment, the second comparison value being a heart rate variability index of the user under the second sound stimulus environment; and
a determination unit configured to determine a possibility that the user has a psychiatric disorder based on the first reference value, the second reference value, the first comparison value, and the second comparison value that are acquired.

A twenty-sixth aspect provides the determination device according to the twenty-fifth aspect, wherein
the first reference value, the second reference value, the first comparison value, and the second comparison value each are a value calculated based on at least one of a plurality of indexes including a high-frequency component of a heart rate variability index and a low-frequency component of a heart rate variability index.

A twenty-seventh aspect provides the determination device according to the twenty-sixth aspect, wherein
the first reference value, the second reference value, the first comparison value, and the second comparison value each are LF/HF that is a ratio of the low-frequency component to the high-frequency component.

A twenty-eighth aspect provides the determination device according to any one of the twenty-fifth to twenty-seventh aspects, wherein
the acquisition unit is configured to acquire the heart rate variability index analyzed from a pulse wave or an electrocardiogram.

A twenty-ninth aspect provides the determination device according to any one of the twenty-fifth to twenty-eighth aspects, wherein
the determination unit is configured to determine a possibility that the user has a psychiatric disorder by comparing the first reference value with the first comparison value.

A thirtieth aspect provides the determination device according to any one of the twenty-fifth to twenty-ninth aspects, wherein
the determination unit is configured to determine a possibility that the user has a psychiatric disorder by comparing the second reference value with the second comparison value.

A thirty-first aspect provides the determination device according to any one of the twenty-fifth to thirtieth aspects, wherein
the determination unit is configured to determine a possibility that the user has a psychiatric disorder by comparing a rate of change of the second reference value to the first reference value and a rate of change of the second comparison value to the first comparison value.

A thirty-second aspect provides the determination device according to any one of the twenty-fifth to thirty-first aspects, wherein
under the first sound stimulus environment, no sound stimulus is given or a sound stimulus is given, and
under the second sound stimulus environment, a sound stimulus is given.

A thirty-third aspect provides the determination device according to the thirty-second aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given,
the acquisition unit is configured to acquire a third reference value and a third comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, and
the determination unit is configured to execute comparison processing among the first reference value, the second reference value, the third reference value, the first comparison value, the second comparison value, and the third comparison value to determine a possibility that the user has a psychiatric disorder.

A thirty-fourth aspect provides the determination device according to the thirty-third aspect, wherein
the determination unit is configured to determine a possibility that the user has a psychiatric disorder by comparing the third reference value with the third comparison value.

A thirty-fifth aspect provides the determination device according to the thirty-third or thirty-fourth aspect, wherein
the determination unit is configured to determine a possibility that the user has a psychiatric disorder by comparing a rate of change of the third reference value to the first reference value and a rate of change of the third comparison value to the first comparison value.

A thirty-sixth aspect provides the determination device according to the thirty-second aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given,
the acquisition unit is configured to acquire a fourth reference value and a fourth comparison value in addition to the first reference value, the second reference value, the first comparison value, and the second comparison value, the fourth reference value being a heart rate variability index of the healthy person to whom the sound stimulus of the first frequency and the sound stimulus of the second frequency are given simultaneously, the fourth comparison value being a heart rate variability index of the user to whom the sound stimulus of the first frequency and the sound stimulus of the second frequency are given simultaneously, and
the determination unit is configured to execute comparison processing among the first reference value, the second reference value, the fourth reference value, the first comparison value, the second comparison value, and the fourth comparison value to determine a possibility that the user has a psychiatric disorder.

A thirty-seventh aspect provides the determination device according to the thirty-sixth aspect, wherein
the determination unit is configured to determine a possibility that the user has a psychiatric disorder by comparing the fourth reference value with the fourth comparison value.

A thirty-eighth aspect provides the determination device according to the thirty-sixth or thirty-seventh aspect, wherein
the determination unit is configured to determine a possibility that the user has a psychiatric disorder by comparing a rate of change of the fourth reference value to the first reference value and a rate of change of the fourth comparison value to the first comparison value.

A thirty-ninth aspect provides the determination device according to the thirty-second aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given,
the acquisition unit is configured to acquire a third reference value, a third comparison value, a fourth reference value, and a fourth comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, the fourth reference value being a heart rate variability index of the healthy person to whom the sound stimulus of the first frequency and the sound stimulus of the second frequency are given simultaneously, the fourth comparison value that being a heart rate variability index of the user to whom the sound stimulus of the first frequency and the sound stimulus of the second frequency are given simultaneously, and
the determination unit is configured to execute comparison among the first reference value, the second reference value, the third reference value, the fourth reference value, the first comparison value, the second comparison value, the third comparison value, and the fourth comparison value to determine a possibility that the user has a psychiatric disorder.

A fortieth aspect provides the determination device according to the thirty-ninth aspect, wherein
the determination unit is configured to determine a possibility that the user has a psychiatric disorder by comparing the third reference value with the third comparison value and comparing the fourth reference value with the fourth comparison value.

A forty-first aspect provides the determination device according to the thirty-ninth or fortieth aspect, wherein
the determination unit is configured to determine a possibility that the user has a psychiatric disorder by comparing a rate of change of the third reference value to the first reference value and a rate of change of the third comparison value to the first comparison value, and comparing a rate of change of the fourth reference value to the first reference value and a rate of change of the fourth comparison value to the first comparison value.

A forty-second aspect provides the determination device according to the thirty-second aspect, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the second frequency is given, and
the second comparison value is a heart rate variability index of the user to whom the sound stimulus of the second frequency is given.

A forty-third aspect provides the determination device according to any one of the twenty-fifth to forty-second aspects, wherein
the psychiatric disorder is a state of depressive symptoms, manic symptoms, anxiety symptoms, or hallucinations and delusions.

A forty-fourth aspect provides the determination device according to any one of the twenty-fifth to forty-second aspects, wherein
the psychiatric disorder is a mood disorder, an anxiety disorder, or schizophrenia.

A forty-fifth aspect provides the determination device according to any one of the twenty-fifth to forty-second aspects, wherein
the psychiatric disorder is a mood disorder or schizophrenia.

A forty-sixth aspect provides the determination device according to the forty-fifth aspect, wherein
the mood disorder is depression or a bipolar disorder.

A forty-seventh aspect provides the determination device according to the forty-sixth aspect, wherein
the bipolar disorder is a depressive episode.

A forty-eighth aspect provides a determination system, including:
the determination device according to any one of the twenty-fifth to forty-seventh aspects;
an analysis device configured to analyze a heart rate variability index of the healthy person or the user; and
a stimulation device configured to give a sound stimulus to the healthy person or the user.

An appropriate combination of any embodiments or modifications among the various embodiments or modifications described above can achieve the effects of the respective embodiments or modifications. In addition, any embodiments can be combined, any examples can be combined, or any embodiment is combined with any example. Further, features in different embodiments or different examples can be combined.

Although the present disclosure has been fully described in connection with preferred embodiments with reference to the accompanying drawings, various changes and modifications will be apparent to those skilled in the art. Such variations and modifications are to be understood as being included within the scope of the present disclosure as set forth in the appended claims.

### EXPLANATION OF REFERENCES

- 1: determination system for a psychiatric disorder
- 10: stimulation device
- 20: analysis device
- 30: determination device
- 31: acquisition unit
- 32: determination unit
- 301: CPU
- 302: memory

## Claims

1. A determination method for a psychiatric disorder, comprising:
acquiring a first reference value, a second reference value, a first comparison value, and a second comparison value, the first reference value being a heart rate variability index of a healthy person under a first sound stimulus environment, the second reference value being a heart rate variability index of the healthy person under a second sound stimulus environment different from the first sound stimulus environment, the first comparison value being a heart rate variability index of a user under the first sound stimulus environment, the second comparison value being a heart rate variability index of the user under the second sound stimulus environment; and
executing comparison processing among the first reference value, the second reference value, the first comparison value, and the second comparison value to determine a possibility that the user has a psychiatric disorder.

2. The determination method according to claim 1, wherein
the first reference value, the second reference value, the first comparison value, and the second comparison value each are a value calculated based on at least one of a plurality of indexes including a high-frequency component of a heart rate variability index and a low-frequency component of the heart rate variability index.

3. The determination method according to claim 2, wherein
the first reference value, the second reference value, the first comparison value, and the second comparison value each are LF/HF that is a ratio of the low-frequency component to the high-frequency component.

4. The determination method according to any one of claims 1 to 3, wherein
the heart rate variability index is analyzed from a pulse wave or an electrocardiogram.

5. The determination method according to any one of claims 1 to 4, wherein
a possibility that the user has a psychiatric disorder is determined by comparing the first reference value with the first comparison value.

6. The determination method according to any one of claims 1 to 5, wherein
a possibility that the user has a psychiatric disorder is determined by comparing the second reference value with the second comparison value.

7. The determination method according to any one of claims 1 to 6, wherein
a possibility that the user has a psychiatric disorder is determined by comparing a rate of change of the second reference value to the first reference value and a rate of change of the second comparison value to the first comparison value.

8. The determination method according to any one of claims 1 to 7, wherein
under the first sound stimulus environment, no sound stimulus is given or a sound stimulus is given, and
under the second sound stimulus environment, a sound stimulus is given.

9. The determination method according to claim 8, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given, and
the method comprises:
acquiring a third reference value and a third comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, and
executing comparison processing among the first reference value, the second reference value, the third reference value, the first comparison value, the second comparison value, and the third comparison value to determine a possibility that the user has a psychiatric disorder.

10. The determination method according to claim 9, wherein
a possibility that the user has a psychiatric disorder is determined by comparing the third reference value with the third comparison value.

11. The determination method according to claim 9 or 10, wherein
a possibility that the user has a psychiatric disorder is determined by comparing a ratio of change of the third reference value to the first reference value and a ratio of change of the third comparison value to the first comparison value.

12. The determination method according to claim 8, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given, and
the method comprises:
acquiring a fourth reference value and a fourth comparison value in addition to the first reference value, the second reference value, the first comparison value, and the second comparison value, the fourth reference value being a heart rate variability index of the healthy person to whom the sound stimulus of the first frequency and the sound stimulus of the second frequency are given simultaneously, the fourth comparison value being a heart rate variability index of the user to whom the sound stimulus of the first frequency and the sound stimulus of the second frequency are given simultaneously, and
executing comparison processing among the first reference value, the second reference value, the fourth reference value, the first comparison value, the second comparison value, and the fourth comparison value to determine a possibility that the user has a psychiatric disorder.

13. The determination method according to claim 12, wherein
a possibility that the user has a psychiatric disorder is determined by comparing the fourth reference value with the fourth comparison value.

14. The determination method according to claim 12 or 13, wherein
a possibility that the user has a psychiatric disorder is determined by comparing a rate of change of the fourth reference value to the first reference value and a rate of change of the fourth comparison value to the first comparison value.

15. The determination method according to claim 8, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given, and
the method comprises:
acquiring a third reference value, a third comparison value, a fourth reference value, and a fourth comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, the fourth reference value being a heart rate variability index of the healthy person to whom the sound stimulus of the first frequency and the sound stimulus of the second frequency are given simultaneously, the fourth comparison value that being a heart rate variability index of the user to whom the sound stimulus of the first frequency and the sound stimulus of the second frequency are given simultaneously, and
executing comparison processing among the first reference value, the second reference value, the third reference value, the fourth reference value, the first comparison value, the second comparison value, the third comparison value, and the fourth comparison value to determine a possibility that the user has a psychiatric disorder.

16. The determination method according to claim 15, wherein
a possibility that the user has a psychiatric disorder is determined by comparing the third reference value with the third comparison value and comparing the fourth reference value with the fourth comparison value.

17. The determination method according to claim 15 or 16, wherein
a possibility that the user has a psychiatric disorder is determined by comparing a rate of change of the third reference value to the first reference value and a rate of change of the third comparison value to the first comparison value, and comparing a rate of change of the fourth reference value to the first reference value and a rate of change of the fourth comparison value to the first comparison value.

18. The determination method according to claim 8, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the second frequency is given, and
the second comparison value is a heart rate variability index of the user to whom the sound stimulus of the second frequency is given.

19. The determination method according to any one of claims 1 to 18, wherein
the psychiatric disorder is a state of depressive symptoms, manic symptoms, anxiety symptoms, or hallucinations and delusions.

20. The determination method according to any one of claims 1 to 18, wherein
the psychiatric disorder is a mood disorder, an anxiety disorder, or schizophrenia.

21. The determination method according to any one of claims 1 to 18, wherein
the psychiatric disorder is a mood disorder or schizophrenia.

22. The determination method according to claim 21, wherein
the mood disorder is depression or a bipolar disorder.

23. The determination method according to claim 22, wherein
the bipolar disorder is a depressive episode.

24. A program for causing a computer to execute the determination method according to any one of claims 1 to 23.

25. A determination device for a psychiatric disorder, comprising:
an acquisition unit configured to acquire a first reference value, a second reference value, a first comparison value, and a second comparison value, the first reference value being a heart rate variability index of a healthy person under a first sound stimulus environment, the second reference value being a heart rate variability index of the healthy person under a second sound stimulus environment, the first comparison value being a heart rate variability index of a user under the first sound stimulus environment, the second comparison value being a heart rate variability index of the user under the second sound stimulus environment; and
a determination unit configured to determine a possibility that the user has a psychiatric disorder based on the first reference value, the second reference value, the first comparison value, and the second comparison value that are acquired.

26. The determination device according to claim 25, wherein
the first reference value, the second reference value, the first comparison value, and the second comparison value each are a value calculated based on at least one of a plurality of indexes including a high-frequency component of a heart rate variability index and a low-frequency component of a heart rate variability index.

27. The determination device according to claim 26, wherein
the first reference value, the second reference value, the first comparison value, and the second comparison value each are LF/HF that is a ratio of the low-frequency component to the high-frequency component.

28. The determination device according to any one of claims 25 to27, wherein
the acquisition unit is configured to acquire the heart rate variability index analyzed from a pulse wave or an electrocardiogram.

29. The determination device according to any one of claims 25 to 28, wherein
the determination unit is configured to determine a possibility that the user has a psychiatric disorder by comparing the first reference value with the first comparison value.

30. The determination device according to any one of claims 25 to 29, wherein
the determination unit is configured to determine a possibility that the user has a psychiatric disorder by comparing the second reference value with the second comparison value.

31. The determination device according to any one of claims 25 to 30, wherein
the determination unit is configured to determine a possibility that the user has a psychiatric disorder by comparing a rate of change of the second reference value to the first reference value and a rate of change of the second comparison value to the first comparison value.

32. The determination device according to any one of claims 25 to 31, wherein
under the first sound stimulus environment, no sound stimulus is given or a sound stimulus is given, and
under the second sound stimulus environment, a sound stimulus is given.

33. The determination device according to claim 32, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given,
the acquisition unit is configured to acquire a third reference value and a third comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, and
the determination unit is configured to execute comparison processing among the first reference value, the second reference value, the third reference value, the first comparison value, the second comparison value, and the third comparison value to determine a possibility that the user has a psychiatric disorder.

34. The determination device according to claim 33, wherein
the determination unit is configured to determine a possibility that the user has a psychiatric disorder by comparing the third reference value with the third comparison value.

35. The determination device according to claim 33 or 34, wherein
the determination unit is configured to determine a possibility that the user has a psychiatric disorder by comparing a rate of change of the third reference value to the first reference value and a rate of change of the third comparison value to the first comparison value.

36. The determination device according to claim 32, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given,
the acquisition unit is configured to acquire a fourth reference value and a fourth comparison value in addition to the first reference value, the second reference value, the first comparison value, and the second comparison value, the fourth reference value being a heart rate variability index of the healthy person to whom the sound stimulus of the first frequency and the sound stimulus of the second frequency are given simultaneously, the fourth comparison value being a heart rate variability index of the user to whom the sound stimulus of the first frequency and the sound stimulus of the second frequency are given simultaneously, and
the determination unit is configured to execute comparison processing among the first reference value, the second reference value, the fourth reference value, the first comparison value, the second comparison value, and the fourth comparison value to determine a possibility that the user has a psychiatric disorder.

37. The determination device according to claim 36, wherein
the determination unit is configured to determine a possibility that the user has a psychiatric disorder by comparing the fourth reference value with the fourth comparison value.

38. The determination device according to claim 36 or 37, wherein
the determination unit is configured to determine a possibility that the user has a psychiatric disorder by comparing a rate of change of the fourth reference value to the first reference value and a rate of change of the fourth comparison value to the first comparison value.

39. The determination device according to claim 32, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the first frequency is given,
the second comparison value is a heart rate variability index of the user to whom the sound stimulus of the first frequency is given,
the acquisition unit is configured to acquire a third reference value, a third comparison value, a fourth reference value, and a fourth comparison value in addition to the first reference value, the second reference value, the first comparison value, and the second comparison value, the third reference value being a heart rate variability index of the healthy person to whom the sound stimulus of the second frequency is given, the third comparison value being a heart rate variability index of the user to whom the sound stimulus of the second frequency is given, the fourth reference value being a heart rate variability index of the healthy person to whom the sound stimulus of the first frequency and the sound stimulus of the second frequency are given simultaneously, the fourth comparison value that being a heart rate variability index of the user to whom the sound stimulus of the first frequency and the sound stimulus of the second frequency are given simultaneously, and
the determination unit is configured to execute comparison among the first reference value, the second reference value, the third reference value, the fourth reference value, the first comparison value, the second comparison value, the third comparison value, and the fourth comparison value to determine a possibility that the user has a psychiatric disorder.

40. The determination device according to claim 39, wherein
the determination unit is configured to determine a possibility that the user has a psychiatric disorder by comparing the third reference value with the third comparison value and comparing the fourth reference value with the fourth comparison value.

41. The determination device according to claim 39 or 40, wherein
the determination unit is configured to determine a possibility that the user has a psychiatric disorder by comparing a rate of change of the third reference value to the first reference value and a rate of change of the third comparison value to the first comparison value, and comparing a rate of change of the fourth reference value to the first reference value and a rate of change of the fourth comparison value to the first comparison value.

42. The determination device according to claim 32, wherein
a sound stimulus given under the second sound stimulus environment includes a sound stimulus of a first frequency and a sound stimulus of a second frequency higher than the first frequency,
the second reference value is a heart rate variability index of the healthy person to whom the sound stimulus of the second frequency is given, and
the second comparison value is a heart rate variability index of the user to whom the sound stimulus of the second frequency is given.

43. The determination device according to any one of claims 25 to 42, wherein
the psychiatric disorder is a state of depressive symptoms, manic symptoms, anxiety symptoms, or hallucinations and delusions.

44. The determination device according to any one of claims 25 to 42, wherein
the psychiatric disorder is a mood disorder, an anxiety disorder, or schizophrenia.

45. The determination device according to any one of claims 25 to 42, wherein
the psychiatric disorder is a mood disorder or schizophrenia.

46. The determination device according to claim 45, wherein
the mood disorder is depression or a bipolar disorder.

47. The determination device according to claim 46, wherein
the bipolar disorder is a depressive episode.

48. A determination system for a psychiatric disorder, comprising:
the determination device according to any one of claims 25 to 47;
an analysis device configured to analyze a heart rate variability index of the healthy person or the user; and
a stimulation device configured to give a sound stimulus to the healthy person or the user.
